# EUROPEAN PATENT SPECIFICATION

(11) **EP 1 066 059 B1**
(45) Date of publication and mention of the grant of the patent: **15.06.2005**
(21) Application number: 99913822.5
(22) Date of filing: 24.03.1999
(51) Int. Cl.: A61K 47/48, A61K 47/26

(54) **FORMULATIONS FOR PROTECTION OF PEG-INTERFERON ALPHA CONJUGATES**
ZUBEREITUNGEN ZUR STABILISIERUNG VON PEG-INTERFERON ALPHA KONJUGATEN
FORMULATIONS SERVANT A PROTEGER DES CONJUGUES DE PEG ET D'ALPHA-INTERFERON

(30) Priority: 26.03.1998 US 48907
(43) Date of publication of application: 10.01.2001
(73) Proprietor: SCHERING CORPORATION, Kenilworth, New Jersey 07033-0530 (US)
(72) Inventor: KLINE, Douglas, F., New York, NY 10011 (US)
(74) Representative: Naylor, Kathryn May
(86) International application number: PCT/US1999/004268
(87) International publication number: WO 1999/048535

(56) References cited:
- EP-A- 0 420 049
- EP-A- 0 593 868
- EP-A- 0 809 996
- WO-A-95/13090
- WO-A-96/11018
- WO-A-96/24369
- WO-A-97/18832
- WO-A-98/48840

## Description

### Field of the Invention

The present invention pertains to formulations for the stabilization of PEG-interferon alpha conjugates during and after lyophilization, their production and use.

### Background of the Invention

Various natural and recombinant proteins have pharmaceutical utility. Once they have been purified, separated, and formulated, they can be parenterally administered for various therapeutic indications. However, parenterally administered proteins may be immunogenic, may be relatively water insoluble, and may have a short pharmacological half life. Consequently, it can be difficult to achieve therapeutically useful blood levels of the proteins in patients.

These problems can be overcome by conjugating the proteins to polymers, such as polyethylene glycol. Davis *et al*., U.S. Pat. No. 4,179,337 disclose conjugating polyethylene glycol (PEG) to proteins such as enzymes and insulin to obtain conjugates having less immunogenic effect than the original proteins and yet still retaining a substantial proportion of their physiological activity. Veronese *et al*., (Applied Biochem. and Biotech, 11:141-152 , 1985) disclose - activating polyethylene glycols with phenyl chloroformates to modify a ribonuclease and a superoxide dimutase. Katre *et al*. U.S. Pat. Nos. 4,766,106 and 4,917,888 also disclose solubilizing proteins by polymer conjugation. Likewise, PEG and other polymers can be conjugated to recombinant proteins to reduce immunogenicity and increase half-life. *See* Nitecki, et al., U.S. Pat. No. 4,902,502, Enzon, Inc., International Application No. PCT/US90/02133, Nishimura *et al*., European Patent Application 154,316 and Tomasi, International Application Number PCT/US85/02572. For example, interferon alpha-2b is known to be effective for treatment of disease states such as renal cell carcinoma, AIDS-related Kaposi's sarcoma, chronic and acute hepatitis B, chronic and acute non-A, non-B/C hepatitis and hepatitis C. Improvement of the pharmacological half-life of interferon alpha-2b would improve treatment of these conditions.

While preparation of protein-polymer conjugates is beneficial, they cannot be used in a practical manner unless they can be stored for an extended period of time during manufacture and distribution to health care providers. Some protein-polymer conjugates, however, rapidly deteriorate, even in frozen solutions. Lyophilization (also known as freeze-drying) is a process that can render a pharmaceutical in a form that can overcome this deficiency.

Lyophilization is a process whereby water is sublimed from a composition after it is frozen. In this process, pharmaceuticals and biologicals that are relatively unstable in an aqueous solution over a period of time can be placed into dosage containers in an easily processed liquid state, dried without the use of damaging heat and stored in a dried state for extended periods.

Due to the low total mass of active substance in each dose, the formulations of most pharmaceuticals and biologicals, including protein-polymer conjugates, require additional ingredients to protect the active ingredient during the lyophilization process. For example, a pharmaceutical filled into a dosage container as a low-concentration aqueous solution can be susceptible to physical loss during the lyophilization vacuum process or adsorption to the container. A lyophilized formulation often contains bulking ingredients that increase the amount of solid material, as well as cryoprotectants, lyoprotectants and other stabilizers to protect the active component from damage. Which particular formulation will protect a given type of pharmaceutical, however, must be determined empirically.

There is a present need for a formulation suitable to protect protein-polymer conjugates, and in particular PEG-interferon alpha conjugates, from damage during lyophilization. Such a formulation should allow PEG-interferon alpha-polymer conjugates to maintain their biological activity, physical stability and chemical stability over extended periods of time.

### Summary of the Invention

The present invention provides formulations that permit stabilization of PEG-interferon alpha conjugates during and after lyophilization.

In a first aspect, the present invention provides a formulation that permits stabilization of PEG-interferon alpha conjugates during and after lyophilization, comprising polyethylene glycol(PEG)-interferon alpha conjugates, a buffer, a stabilizer, a cryoprotectant and a solvent, wherein said cryoprotectant is sucrose. The present invention also contemplates processes for preparing stable, aqueous formulation solutions comprising admixing an effective amount of PEG-interferon alpha conjugates with a buffer, a stabilizer, a cryoprotectant and a solvent wherein said cryoprotectant is sucrose. In a preferred aspect of the process of the present invention, the formulation is prepared and maintained substantially free of dissolved oxygen and a head space of inert atmosphere above the formulation is maintained at a value of less than about 4% oxygen by volume.

The present invention is not limited to specific chemicals for the solution components. However, in a preferred embodiment, the buffer is sodium phosphate, the stabilizer is a poly(oxy-1,2-ethanediyl) derivative, the cryoprotectant is sucrose and the solvent is water. In such an embodiment, the sodium phosphate can comprise sodium phosphate dibasic anhydrous with sodium phosphate monobasic dihydrate.

The present invention is also not limited by the concentrations of the components of the formulations of the present invention. In one embodiment, the concentration of PEG-interferon alpha conjugates is preferably 0.03 to 2.0 mg interferon alpha per ml, while the concentration of sodium phosphate is preferably 0.005 to 0.1 molar, the concentration of poly(oxy-1,2-ethanediyl) derivative is preferably 0.01 to 1.0 mg/ml and the concentration of sucrose is preferably 20 to 100 mg/ml. In a particularly preferred embodiment, the mass of PEG-interferon conjugates is 0.1 mg of - interferon alpha, the mass of sodium phosphate dibasic is 0.75 mg, the mass of sodium phosphate monobasic dihydrate is 0.75 mg, the mass of sucrose is 40 mg, the mass of poly(oxy-1,2-ethanediyl) derivative is 0.05 mg and the volume of water is 0.5 ml. Alternatively, the ratio of components is 0.08% of said PEG-interferon alpha conjugates as measured by the mass of the interferon alpha, 3.6% of sodium phosphate, 0.12% of poly(oxy-1,2-ethanediyl) derivative and 96.2% of sucrose, by weight

While the present invention is not limited to a specific PEG-interferon alpha conjugate, in one embodiment, the PEG-interferon alpha conjugates comprise single PEG molecules conjugated to single interferon molecules. In such an embodiment, the interferon alpha molecules can be selected from the group consisting of interferon alpha-2a, interferon alpha-2b, interferon alpha-2c and consensus interferon. In a preferred embodiment, the interferon molecules are interferon alpha-2b. Likewise, while the present invention is not limited to a specific PEG molecule, in one embodiment, the polyethylene glycol is PEG₁₂₀₀₀. In a particularly preferred embodiment, the interferon alpha-2b molecules are linked to the PEG₁₂₀₀₀ molecules with a urethane bond.

While not limited to a specific characterization, when single interferon alpha molecules are linked to single polymer molecules, the present invention contemplates that the resulting PEG-interferon alpha conjugates can comprise a mixture of positional isomers. In a preferred embodiment, one of the positional isomers is an interferon alpha-2b molecule linked to a PEG₁₂₀₀₀ molecule at a histidine residue on the interferon alpha-2b molecule.

The present invention also contemplates a process of lyophilization, comprising lyophilization of the formulations described above to create a lyophilized powder. In a preferred embodiment, the process further comprises reconstitution of the lyophilized powder with water or other aqueous diluents, such as benzyl alcohol-containing bacteriostatic water for injection, to create a reconstituted solution (Bacteriostatic Water for Injection, Abbott Laboratories, Abbott Park, IL).

The present invention also contemplates lyophilized powders produced by lyophilization of the formulations described above. In a preferred embodiment, the lyophilized powder comprises 0.08% of said PEG-interferon alpha conjugates, 3.6% of said sodium phosphate, 0.12% of said poly(oxy-1,2-ethanediyl) derivative and 96.2% of said sucrose, by weight.

Likewise, articles of manufacture comprising a syringe or a vial containing an effective amount of such lyophilized powders is contemplated. In a preferred embodiment, the article of manufacture further comprises a volume of water for reconstitution of the powder. In a particularly preferred embodiment, the powder is reconstituted with bacteriostatic water. In a further preferred embodiment, the lyophilized powder is reconstituted with the same volume of water as was removed from the lyophilization solution during lyophilization.

The present invention also contemplates processes for treating diseases in animals. In one embodiment, this process comprises the introduction of the reconstituted solution into an animal having a disease. In one embodiment, the animal is human. In a preferred embodiment, the human is infected with a hepatitis virus, such as hepatitis C virus. In an alternate preferred embodiment, the human has cancer.

### Detailed Description of the Invention

"PEG-interferon alpha conjugates" are interferon alpha molecules covalently attached to a PEG molecule. In preferred embodiments, the PEG-interferon alpha conjugates of the present invention comprises interferon alpha-2a (Roferon, Hoffman La-Roche, Nutley, NJ), interferon alpha 2b (Intron, Schering-Plough, Madison, NJ), interferon alpha-2c (Berofor Alpha, Boehringer Ingelheim, Ingelheim, Germany) or consensus interferon as defined by determination of a consensus sequence of naturally occurring interferon alphas (Infergen, Amgen, Thousand Oaks, CA).

Polymers, on the other hand, are molecules having covalently attached repeating chemical units. Often, the approximate molecular weight of the polymer is designated with a number following the name of the repeated chemical unit. For example, "PEG₁₂₀₀₀" or "polyethylene glycol (12,000)" refers to a polymer of polyethylene glycol having an average molecular weight of approximately 12,000. In a PEG₁₂₀₀₀ polymer, the number of repeated polyethylene glycol units in the polymer is approximately 273. It is understood that these designations are approximate as polymers are manufactured in the form of a mixture having a distribution of chain lengths, giving an average molecular weight, and it is often impossible to manufacture a polymer having a precise and uniform molecular weight or number of repeated units. Various other polymers and their methods for production are well known in the art.

Methods for creating protein-polymer conjugates are also known in the art. For example, U.S. Patent 5,691,154 to Callstrom et al, U.S. Patent No. 5,686,071 to Subramanian et al, U.S. Patent No. 5,639.633 to Callstrom et al, U.S. Patent No. 5,492,821 to Callstrom et al, U.S. Patent No. 5,447,722 to Lang et al and U.S. Patent No. 5,091,176 to Braatz et al all provide methods for producing protein-polymer conjugates.

Conjugation of polymers to proteins may result in a single polymer molecule conjugated to a protein or multiple such conjugations to a single protein. The degree of conjugation is dependent upon the reaction conditions and desired result. In a preferred embodiment, the PEG-interferon alpha conjugate in the formulations of the present invention comprises a single interferon alpha-2b conjugated to a single PEG₁₂₀₀₀. In a particularly preferred embodiment, the interferon alpha-2b molecule is linked to the PEG₁₂₀₀₀ molecule with a urethane bond. Reagents and methods for producing this protein-polymer conjugate can be found in U.S. Patent No. 5,612,460 to Zalipsky and U.S. Patent No. 5,711,944 to Gilbert, et al. When such a protein-polymer conjugate is utilized in the formulation solutions of the present invention, the preferred concentration of PEG-interferon alpha conjugate is 0.03 to 2.0 mg interferon alpha per ml.

When a single interferon alpha molecule is linked to a single polymer molecule, the resulting PEG-interferon alpha conjugates may be in the form of a single positional isomer or in a mixture of positional isomers. A "mixture of positional isomers" indicates that the individual PEG-interferon alpha conjugates may be linked at different sites on different interferon alpha molecules. For example, in one embodiment of the present invention, the PEG-interferon alpha mixture contains at least one PEG-interferon alpha conjugate linked at a histidine residue of the interferon alpha molecule, while another PEG-interferon alpha conjugate is linked at another site of the interferon alpha molecule (e.g. the amino terminus).

As described above, preservation of PEG-interferon alpha conjugates can be achieved by lyophilization. Lyophilization is a process of freeze-drying a composition wherein a frozen aqueous mixture is treated to remove water. Commonly, the process involves the sublimation of water from the frozen aqueous solutions, usually under reduced pressure conditions. After lyophilization, the PEG-interferon alpha conjugate can be stored for extended periods of time.

PEG-interferon alpha conjugates, however, are subject to damage during and after lyophilization. Damage to PEG-interferon alpha conjugates can be characterized by the loss of protein, loss of biological activity or by the change in the degree and/or nature of conjugation of the interferon alpha. For example, a PEG-interferon alpha conjugate may degrade into free PEG and interferon alpha, resulting in a lowering of the degree of conjugation. Likewise, the resulting free PEG may become available to conjugate to another interferon alpha, potentially resulting in the increase of the degree of conjugation in that target molecule. Similarly, a PEG-interferon alpha conjugate may undergo an intramolecular shift of the PEG from one site of conjugation to another within the same molecule, thereby changing the nature of conjugation of the interferon alpha.

The present invention protects PEG-interferon alpha conjugates from damage by including them in formulations that prevent damage during and after lyophilization. While the present invention is not limited to a particular formulation, in a preferred embodiment, the method utilizes a buffer, stabilizer, cryoprotectant and solvent, in addition to the PEG-interferon alpha conjugate.

Buffers are suitable for maintaining the pH of the formulation in a range of 4.5 to 7.1, preferably 6.5-7.1 and most preferably 6.8. The use of a buffer system of sodium phosphate dibasic and sodium phosphate monobasic is preferred. When a sodium phosphate dibasic anhydrous/monobasic dihydrate system is utilized, it is preferably in equal mass amounts of dibasic to monobasic at a preferred total concentration of 0.005 to 0.1 molar. Other suitable buffer systems to maintain the desired pH range include sodium citrate/citric acid and sodium acetate/acetic acid.

A stabilizing agent is useful to prevent adsorption of the PEG-interferon alpha conjugate to the stainless steel and glass surfaces of the equipment used to make and store the formulations containing the PEG-interferon alpha conjugate. As one example, poly(oxy-1,2-ethanediyl) derivatives are useful as stabilizing agents. Mono-9-octadecenoate poly(oxy-1,2-ethanediyl) derivatives (Polysorbate 80) is a preferred stabilizing agent. When polysorbate 80 is utilized, the preferred concentration is 0.01 to 1 mg/ml.

Cryoprotectants, also known as cryoprotective agents or compounds, are agents that protect chemical compounds, cells, or tissues from the deleterious effects of freezing, such as that usually accompanying lyophilization. In the case of PEG-interferon alpha conjugates, cryoprotectants can protect them from damage, adsorption and loss from vacuum utilized in lyophilization.

The cryoprotectant is sucrose.

Likewise, the present invention is not limited to any particular amount of cryoprotectant used. In one embodiment, cryoprotectants are present in an amount sufficient to allow the PEG-interferon alpha conjugate to be lyophilized. In such an embodiment, cryoprotectants can be present in an amount of 0.05% to 90%, preferably 0.05-50%, and most preferably in an amount of about 0.15% to about 10%, based on the total weight of the PEG-interferon alpha solution. When sucrose is used, the preferred concentration is 20 to 100 mg/ml.

Formulations including an effective amount of biologically active PEG-interferon alpha conjugates are useful in treating disease states, preferably as injectable aqueous solutions. An effective amount means the formulation or powder has an adequate concentration of biologically active component to treat a disease state in an animal. For example, the preferred interferon alpha-2b-PEG₁₂₀₀₀ conjugates are suitable for treatment of disease states such as renal cell carcinoma, AIDS-related Kaposi's sarcoma, chronic and acute hepatitis B, chronic and acute non-A, non-B/C hepatitis and hepatitis C. One solution containing an effective amount of this PEG-interferon alpha conjugate contains 0.03 to 2.0 mg/ml of PEG₁₂₀₀₀-interferon alpha-2b conjugate as measured by protein mass.

### Example

This example provides a description of a formulation of the present invention and protection of one PEG-interferon alpha conjugate during lyophilization and storage. The PEG-interferon alpha conjugate is introduced in a lyophilization formulation, lyophilized and stored as a dry powder. The components of the formulation are as follows:

**Table 1:**

| **Formulation for Lyophilization and Storage** | |
|---|---|
| Component | mg/vial* |
| interferon alfa-2b-PEG₁₂₀₀₀ | 0.1*** |
| Sodium Phosphate Dibasic Anhydrous | 0.75 |
| Sodium Phosphate Monobasic Dihydrate | 0.75 |
| Sucrose | 40 |
| Polysorbate 80 | 0.05 |
| Water for Injection (q.s. ad) | 0.5 ml** |

| | |
|---|---|
| *Amount contained in label volume of 0.5 ml | |
| **Water is sublimed during lyophilization. | |
| ***Based on protein mass. | |

After lyophilization, the resulting powder is stored and, over a period of six months, samples are reconstituted with water for analysis. The reconstituted solution is analyzed for protein mass content, degree of conjugation of the PEG-interferon alpha conjugate, bioactivity and visual clarity. The results are present in Table 2.

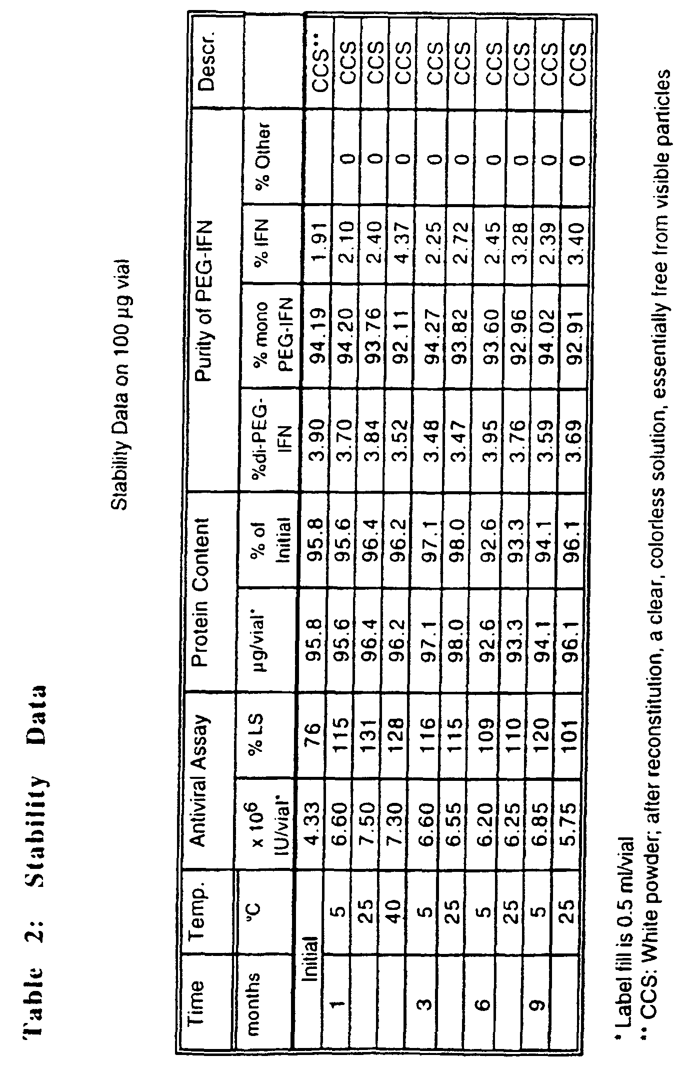

The results show that the total protein mass content is relatively stable over the nine-month period. Additionally, the change in degree of the monopegylated interferon alfa-2b (*i.e.*, degradation to free interferon and polymer or creation of dipegylated interferon) negligible. The bioactivity as measured by-a cell-based antiviral assay remains essentially unchanged. The reconstituted solutions remain clear, colorless and free from visible particles throughout the six-month period. This demonstrates a surprisingly high stability during lyophilization and subsequent storage.

From the above, it is clear that the present invention provides formulations suitable to protect PEG-interferon alpha conjugates from damage during lyophilization and during subsequent storage.

## Claims

1. An aqueous formulation that permits stabilization of PEG-interferon alpha conjugates during and after lyophilization comprising polyethylene glycol (PEG)-interferon alpha conjugates, a buffer, a stabilizer, a cryoprotectant and a solvent, wherein said cryoprotectant is sucrose.

2. The formulation of claim 1, wherein said stabilizer is a poly(oxy-1,2-ethanediyl) derivative.

3. The formulation of claim 1, wherein said buffer is selected from the group consisting of a sodium phosphate dibasic/sodium phosphate monobasic buffer, a sodium citrate/citric acid buffer and a sodium acetate/acetic acid buffer.

4. The formulation of claim 2, wherein said buffer is sodium phosphate and said solvent is water.

5. The formulation of claim 4, wherein said sodium phosphate comprises sodium phosphate dibasic anhydrous and sodium phosphate monobasic dihydrate.

6. The formulation of claim 2, wherein said poly(oxy-1,2-ethanediyl) derivative is polysorbate 80.

7. The formulation of claim 1, wherein said PEG-interferon alpha conjugates comprise single PEG molecules conjugated to single interferon alpha molecules.

8. The formulation of claim 7, wherein said interferon alpha molecules are selected from the group consisting of interferon alpha-2a, interferon alpha-2b, interferon alpha-2c and consensus interferon.

9. The formulation of claim 7, wherein said PEG is PEG₁₂₀₀₀.

10. The formulation of claim 8, wherein said interferon alpha molecules are interferon alpha-2b.

11. The formulation of claim 10, wherein said PEG molecules are PEG₁₂₀₀₀, and wherein said interferon alpha-2b molecules are linked to said PEG₁₂₀₀₀ molecules with a urethane bond.

12. The formulation of claim 7, wherein said PEG-interferon alpha conjugates comprise a mixture of positional isomers.

13. The formulation of claim 12, wherein one of said positional isomers comprises an interferon alpha-2b molecule linked to a PEG₁₂₀₀₀ molecule at a histidine residue on said interferon alpha-2b molecule.

14. The formulation of claim 4, wherein the concentration of said PEG-interferon alpha conjugates is 0.03 to 2.0 mg interferon alpha per ml, the concentration of said sodium phosphate is 0.005 to 0.1 molar, the concentration of said poly(oxy-1,2-ethanediyl) derivative is 0.01 to 1.0 mg/ml, and the concentration of said sucrose is 20 to 100 mg/ml.

15. The formulation of claim 5, wherein the mass of said PEG-interferon alpha conjugates is 0.1 mg of interferon alpha, the mass of said sodium phosphate dibasic anhydrous is 0.75 mg, the mass of said sodium phosphate monobasic dihydrate is 0.75 mg, the mass of said sucrose is 40 mg, the mass of said poly(oxy-1,2-ethanediyl) derivative is 0.05 mg and the volume of said water is 0.5 ml.

16. The formulation of any preceding claim, wherein the said PEG-interferon alpha conjugate is PEG₁₂₀₀₀-interferon alpha-2b conjugate.

17. An aqueous formulation that permits stabilization of PEG-interferon alpha conjugates during and after lyophilization comprising interferon alpha-2b-PEG₁₂₀₀₀, sodium phosphate dibasic anhydrous, sodium phosphate monobasic dihydrate, sucrose, polysorbate 80, and water, wherein the mass of said interferon alpha-2b-PEG₁₂₀₀₀ is 0.1 mg of interferon alpha, the mass of said sodium phosphate dibasic anhydrous is 0.75 mg, the mass of said sodium phosphate monobasic dihydrate is 0.75 mg, the mass of said sucrose is 40 mg, the mass of said polysorbate 80 is 0.05 mg and the volume of said water is 0.5 ml.

18. A process for producing a lyophilized powder comprising lyophilizing the formulation of any one of claims 1-17.

19. The lyophilized powder producible by the method of claim 18.

20. A lyophilized powder of claim 19, wherein said powder comprises 0.08% of said PEG-interferon alpha conjugates as measured by the mass of the interferon alpha, 3.6% of said sodium phosphate, 0.12% of said poly(oxy-1,2-ethanediyl) derivative and 96.2% of said sucrose, by weight.

21. A process for producing a solution of PEG-interferon alpha conjugates comprising the steps of:
a) providing said lyophilized powder of claim 19; and
b) reconstituting said lyophilized powder with water to create said solution.

22. The process of claim 21, wherein said water comprises bacteriostatic water.

23. An article of manufacture comprising a syringe or a vial containing said lyophilized powder of either one of claims 19 or 20.

24. The article of manufacture of claim 23, further comprising a volume of water for reconstitution of said powder.

25. The article of manufacture of claim 24, wherein said water comprises bacteriostatic water.

## Patentansprüche

1. Wässrige Formulierung, die Stabilisierung von PEG-Interferon α-Konjugaten während und nach Lyophilisierung ermöglicht, die Polyethylenglykol- (PEG)-Interferon α-Konjugate, einen Puffer, einen Stabilisator, ein Kryoschutzmittel und ein Lösungsmittel enthält, wobei das Kryoschutzmittel Sucrose ist.

2. Formulierung nach Anspruch 1, bei der der Stabilisator ein Poly(oxy-1,2-ethandiyl)derivat ist.

3. Formulierung nach Anspruch 1, bei der der Puffer ausgewählt ist aus der Gruppe bestehend aus einem Dinatriumphosphat/Mononatriumphosphat-Puffer, einem Natriumcitrat/Citronensäure-Puffer und einem Natriumacetat/Essigsäure-Puffer.

4. Formulierung nach Anspruch 2, bei der der Puffer Natriumphosphat ist und das Lösungsmittel Wasser ist.

5. Formulierung nach Anspruch 4, bei der das Natriumphosphat wasserfreies Dinatriumphosphat und Mononatriumphosphatdihydrat enthält.

6. Formulierung nach Anspruch 2, bei der das Poly(oxy-1,2-ethandiyl)derivat Polysorbat 80 ist.

7. Formulierung nach Anspruch 1, bei der die PEG-Interferon α-Konjugate einzelne PEG-Moleküle enthalten, die mit einzelnen Interferon α-Molekülen konjugiert sind.

8. Formulierung nach Anspruch 7, bei der die Interferon α-Moleküle ausgewählt sind aus der Gruppe bestehend aus Interferon α-2a, Interferon α-2b, Interferon α-2c und Consensus-Interferon.

9. Formulierung nach Anspruch 7, bei der das PEG PEG₁₂₀₀₀ ist.

10. Formulierung nach Anspruch 8, bei der die Interferon α-Moleküle Interferon α-2b sind.

11. Formulierung nach Anspruch 10, bei der die PEG-Moleküle PEG₁₂₀₀₀ sind, und bei der die Interferon α-2b-Moleküle über eine Urethanbindung an die PEG₁₂₀₀₀-Moleküle gebunden sind.

12. Formulierung nach Anspruch 7, bei der die PEG-Interferon α-Konjugate eine Mischung von Positionsisomeren enthalten.

13. Formulierung nach Anspruch 12, bei der eines der Positionsisomere ein Interferon α-2b-Molekül enthält, das an einem Histidinrest an dem Interferon α-2b-Molekül an ein PEG₁₂₀₀₀-Molekül gebunden ist.

14. Formulierung nach Anspruch 4, bei der die Konzentration der PEG-Interferon α-Konjugate 0,03 bis 2,0 mg Interferon α pro ml beträgt, die Konzentration des Natriumphosphats 0,005 bis 0,1 molar ist, die Konzentration des Poly(oxy-1,2-ethandiyl)derivats 0,01 bis 1,0 mg/ml ist und die Konzentration der Sucrose 20 bis 100 mg/ml ist.

15. Formulierung nach Anspruch 5, bei der die Masse der PEG-Interferon α-Konjugate 0,1 mg Interferon α ist, die Masse des wasserfreien Dinatriumphosphats 0,75 mg ist, die Masse des Mononatriumphosphatdihydrats 0,75 mg ist, die Masse der Sucrose 40 mg ist, die Masse des Poly(oxy-1,2-ethandiyl)derivats 0,05 mg ist und das Volumen des Wassers 0,5 ml ist.

16. Formulierung nach einem der vorhergehenden Ansprüche, bei der das PEG-Interferon α-Konjugat PEG₁₂₀₀₀-Interferon α-2b-Konjugat ist.

17. Wässrige Formulierung, die Stabilisierung von PEG-Interferon α-Konjugaten während und nach der Lyophilisierung ermöglicht, die Interferon α-2b-PEG₁₂₀₀₀, wasserfreies Dinatriumphosphat, Mononatriumphosphatdihydrat, Sucrose, Polysorbat 80 und Wasser enthält, wobei die Masse des Interferon α-2b-PEG₁₂₀₀₀ 0,1 mg Interferon α ist, die Masse des wasserfreien Dinatriumphosphats 0,75 mg ist, die Masse des Mononatriumphosphatdihydrats 0,75 mg ist, die Masse der Sucrose 40 mg ist, die Masse des Polysorbats 80 0,05 mg ist und das Volumen des Wassers 0,5 ml ist.

18. Verfahren zur Herstellung eines lyophilisierten Pulvers, bei dem die Formulierung gemäß einem der Ansprüche 1 bis 17 lyophilisiert wird.

19. Lyophilisiertes Pulver, das nach dem Verfahren von Anspruch 18 herstellbar ist.

20. Lyophilisiertes Pulver nach Anspruch 19, bei dem das Pulver 0,08 Gew.-% der PEG-Interferon α-Konjugate, gemessen durch die Masse des Interferon α, 3,6 % des Natriumphosphats, 0,12 Gew.-% des Poly(oxy-1,2-ethandiyl)derivats und 96,2 Gew.-% der Sucrose enthält.

21. Verfahren zur Herstellung einer Lösung von PEG-Interferon α-Konjugaten, umfassend die Schritte:
a) Bereitstellen des lyophilisierten Pulvers von Anspruch 19 und
b) Wiederauflösen des lyophilisierten Pulvers mit Wasser, um die Lösung zu erzeugen.

22. Verfahren nach Anspruch 21, bei dem das Wasser bakteriostatisches Wasser umfasst.

23. Gegenstand, der eine Spritze oder eine Ampulle umfasst, die das lyophilisierte Pulver nach einem der Ansprüche 19 oder 20 enthält.

24. Gegenstand nach Anspruch 23, der ferner ein Volumen an Wasser zum Wiederauflösen des Pulvers enthält.

25. Gegenstand nach Anspruch 24, bei dem das Wasser bakteriostatisches Wasser umfasst.

## Revendications

1. Préparation aqueuse permettant de stabiliser les conjugués PEG-interféron alpha pendant et après la lyophilisation, qui comprend des conjugués polyéthylèneglycol (PEG)-interféron alpha, un tampon, un stabilisant, un cryoprotecteur et un solvant, ledit cryoprotecteur étant du sucrose.

2. Préparation selon la revendication 1, dont ledit stabilisant est un dérivé poly(oxy-1,2-éthanediyl).

3. Préparation selon la revendication 1, dont ledit tampon est un tampon choisi parmi un tampon phosphate monosodique/phosphate disodique, un tampon acide citrique/citrate de sodium et un tampon acide acétique/acétate de sodium.

4. Préparation selon la revendication 2, dont ledit tampon est du phosphate de sodium et ledit solvant est de l'eau.

5. Préparation selon la revendication 4, dont ledit phosphate de sodium comprend du phosphate disodique anhydre et du phosphate monosodique dihydraté.

6. Préparation selon la revendication 2, dont ledit dérivé poly(oxy-1,2-éthanediyl) est du polysorbate 80.

7. Préparation selon la revendication 1, dont lesdits conjugués PEG-interféron alpha comprennent des molécules d'un seul PEG conjuguées avec des molécules d'un seul interféron alpha.

8. Préparation selon la revendication 7, dont lesdites molécules d'interféron alpha sont choisies parmi l'interféron alpha-2a, l'interféron alpha-2b, l'interféron alpha-2c et l'interféron consensus.

9. Préparation selon la revendication 7, dont ledit PEG est du PEG₁₂₀₀₀.

10. Préparation selon la revendication 8, dont lesdites molécules d'interféron alpha sont des molécules d'interféron alpha-2b.

11. Préparation selon la revendication 10, pour laquelle lesdites molécules de PEG sont du PEG₁₂₀₀₀ et lesdites molécules d'interféron alpha-2b sont liées auxdites molécules de PEG₁₂₀₀₀ par l'intermédiaire d'une liaison uréthane.

12. Préparation selon la revendication 7, dont lesdits conjugués PEG-interféron alpha comprennent un mélange d'isomères de position.

13. Préparation selon la revendication 12, pour laquelle l'un desdits isomères de position comprend une molécule d'interféron alpha-2b liée à une molécule de PEG₁₂₀₀₀, à un reste histidine de ladite molécule d'interféron alpha-2b.

14. Préparation selon la revendication 4, pour laquelle la concentration desdits conjugués PEG-interféron alpha est de 0,03 à 2,0 mg d'interféron alpha/ml, la concentration dudit phosphate de sodium est 0,005 à 0,1 molaire, la concentration dudit dérivé poly(oxy-1,2-éthanediyl) est de 0,01 à 1,0 mg/ml, et la concentration dudit sucrose est de 20 à 100 mg/ml.

15. Préparation selon la revendication 5, dans laquelle la masse desdits conjugués PEG-interféron alpha est de 0,1 mg, comptée en interféron alpha, la masse dudit phosphate disodique anhydre est de 0,75 mg, la masse dudit phosphate monosodique dihydraté est de 0,75 mg, la masse dudit sucrose est de 40 mg, la masse dudit dérivé poly(oxy-1,2-éthanediyl) est de 0,05 mg, et le volume d'eau est de 0,5 ml.

16. Préparation selon l'une quelconque des revendications précédentes, dont ledit conjugué PEG-interféron alpha est un conjugué PEG₁₂₀₀₀-interféron alpha-2b.

17. Préparation aqueuse permettant la stabilisation des conjugués PEG-interféron alpha pendant et après la lyophilisation, qui comprend de l'interféron alpha-2b-PEG₁₂₀₀₀, du phosphate disodique anhydre, du phosphate monosodique dihydraté, du sucrose, du polysorbate 80 et de l'eau, préparation dans laquelle la masse dudit interféron alpha-2b-PEG₁₂₀₀₀ est de 0,1 mg, comptée en interféron alpha, la masse dudit phosphate disodique anhydre est de 0,75 mg, la masse dudit phosphate monosodique dihydraté est de 0,75 mg, la masse dudit sucrose est de 40 mg, la masse dudit polysorbate 80 est de 0,05 mg, et le volume d'eau est de 0,5 ml.

18. Procédé de production d'une poudre lyophilisée, qui comprend la lyophilisation d'une préparation selon l'une quelconque des revendications 1 à 17.

19. Poudre lyophilisée que l'on peut produire par le procédé de la revendication 18.

20. Poudre lyophilisée selon la revendication 19, qui renferme 0,08 % desdits conjugués PEG-interféron alpha, ce pourcentage étant basé sur la masse d'interféron alpha, 3,6 % dudit phosphate de sodium, 0,12 % dudit dérivé poly(oxy-1,2-éthanediyl) et 96,2 % dudit sucrose, les pourcentages étant des pourcentages en poids.

21. Procédé de production d'une solution de conjugués PEG-interféron alpha, qui comprend les étapes consistant à :
a) prendre ladite poudre lyophilisée de la revendication 19, et
b) recombiner ladite poudre lyophilisée avec de l'eau pour former ladite solution.

22. Procédé selon la revendication 21, dans lequel ladite eau est de l'eau bactériostatique.

23. Article manufacturé comprenant une seringue ou une fiole contenant ladite poudre lyophilisée selon la revendication 19 ou 20.

24. Article manufacturé selon la revendication 23, qui comprend en outre un certain volume d'eau pour la reconstitution d'une solution à partir de ladite poudre.

25. Article manufacturé selon la revendication 24, pour lequel ladite eau est de l'eau bactériostatique.
